# EUROPEAN PATENT SPECIFICATION

(11) **EP 3 126 044 B1**
(45) Date of publication and mention of the grant of the patent: **01.08.2018**
(21) Application number: 15715916.1
(22) Date of filing: 24.03.2015
(51) Int. Cl.: B01J 8/18, C07C 253/26, C07C 255/08, B01J 19/26, B01J 4/00

(54) **PROCESS FOR SUPPLYING AMMONIA TO AN AMMOXIDATION REACTOR WITH A SPARGER**
VERFAHREN FÜR DIE ZUFÜHRUNG VON AMMONIAK ZU EINEM AMMOXIDATIONSREAKTOR MIT EINEM GASVERTEILER
PROCÉDÉ POUR L'ALIMENTATION D'AMMONIAC À UN RÉACTEUR D'AMMOXYDATION AVEC UN DIFFUSEUR

(30) Priority: 31.03.2014 CN 201410124747
(43) Date of publication of application: 08.02.2017
(73) Proprietor: Ineos Europe AG, 1180 Rolle (Vaud) (CH)
(72) Inventor: MCDONEL, Timothy Robert, Elburn, IL 60119 (US); COUCH, Jay Robert, Naperville, IL 60564 (US); WAGNER, David Rudolph, Naperville, IL 60564 (US); WACHTENDORF, Paul Trigg, Victoria, TX 77904 (US); TRAVERS, Thomas George, Carpentersville, IL 60110 (US)
(74) Representative: King, Alex
(86) International application number: PCT/US2015/022199
(87) International publication number: WO 2015/153192

(56) References cited:
- WO-A1-01/03823
- WO-A1-2005/084790
- DE-A1- 2 707 478
- JP-A- H08 208 583
- US-A1- 2010 216 896

## Description

### Background

In the commercial manufacture of acrylonitrile, propylene, ammonia and oxygen are reacted together according to the following reaction scheme:

CH₂=CH-CH₃ + NH₃ + 3/2O₂ → CH₂=CH-CN + 3H₂O

This process, which is commonly referred to as ammoxidation, is carried out in the gas phase at elevated temperature in the presence of a suitable fluid bed ammoxidation catalyst.

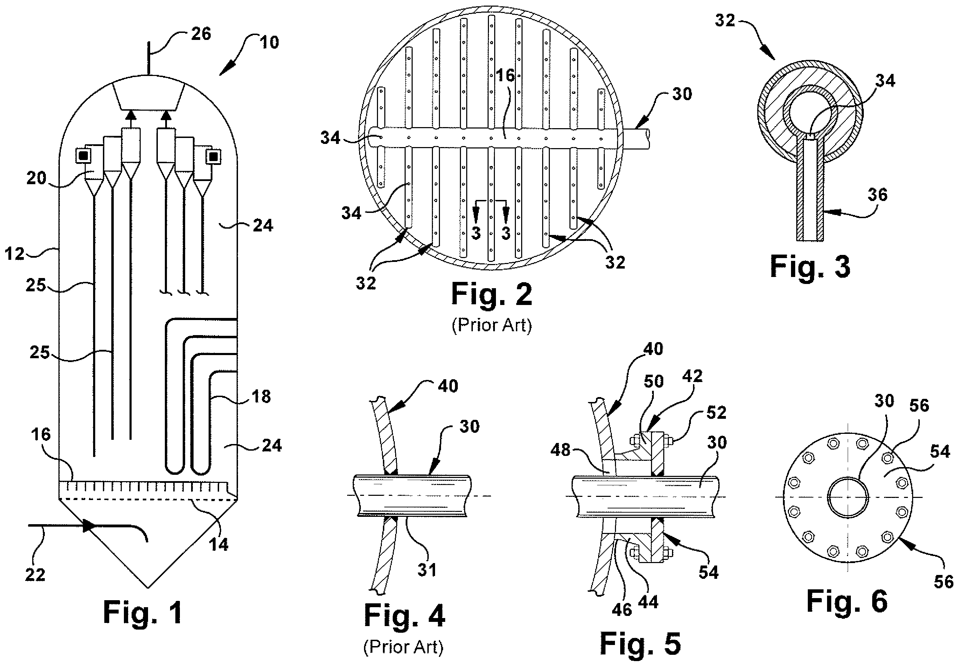

Fig. 1 illustrates a typical ammoxidation reactor used to carry out this process. As shown there, reactor 10 comprises reactor wall 12, air grid 14, feed sparger 16, cooling coils 18 and cyclones 20. During normal operation, process air is charged into reactor 10 through air inlet 22, while a mixture of propylene and ammonia is charged into reactor 10 through feed sparger 16. The flow rates of both are high enough to fluidize a bed 24 of ammoxidation catalyst in the reactor interior, where the catalytic ammoxidation of the propylene and ammonia to acrylonitrile occurs.

Product gases produced by the reaction exit reactor 10 through reactor effluent outlet 26. Before doing so, they pass through cyclones 20, which remove any ammoxidation catalyst these gases may have entrained for return to catalyst bed 24 by diplegs 25. Ammoxidation is highly exothermic, and so cooling coils 18 are used to withdraw excess heat and thereby keep the reaction temperature at an appropriate level.

Propylene and ammonia can form explosive mixtures with oxygen. However, at normal operating temperatures, explosions are prevented inside reactor 10 by the fluidized ammonization catalyst, which preferentially catalyzes the ammoxidation reaction before an explosion can occur. Accordingly, reactor 10 is designed and operated so that the only place process air is allowed to contact propylene and ammonia during normal operation is within the fluidized bed of ammoxidation catalyst 24, and then only when the temperature of the catalyst is high enough to catalyze the ammoxidation reaction.

For this purpose, the traditional way in which propylene and ammonia are fed to reactor 10 uses a feed sparger system 16 such as illustrated in U.S. 5, 256,810. As shown in Figs. 1 and 2 of the '810 patent, which are reproduced as Figs. 2 and 3 of this document, feed sparger 16 takes the form of a series of supply pipes or conduits including main header 30 and laterals 32 attached to and branching out from header 30. A system of downwardly facing feed nozzles 34 is defined in header 30 and laterals 34 through which a mixture of propylene and ammonia is charged during normal reactor operation. The number and spacing laterals 32 and feed nozzles 34 is such that, in the aggregate, about 10 to 30 feed nozzles per square meter are located approximately uniformly across the entire cross-sectional area of reactor 10.

Normally, each feed nozzle 34 is surrounded by a feed shroud 36, which takes the form a short section of conduit having an inside diameter several times larger than the diameter of nozzle 34. Feed shrouds 34 enable the velocity of gas passing out of nozzles 10 to slow considerably before exiting into catalyst bed 24, which prevents disintegration of the catalyst which might otherwise occur.

Process air typically enters catalyst bed 24 (Fig. 1) after passing through air grid 14, which is located below feed sparger 16. As well known, air grid 14 typically takes the form of a continuous metal sheet which defines a series of air holes or nozzles therein. The diameter of the air nozzles, the mass flow rate of process air passing through air grid 14 and the mass flow rate of the propylene/ammonia mixture passing through feed sparger 16 are selected so that the ammoxidation catalyst in catalyst bed 24 is fully fluidized by these gases during normal operation.

Air nozzles are typically provided with their own protective air shrouds (not shown), which are normally located below air grid 14. In addition, in many cases, feed nozzles 34 are provided in a one-to-one relationship with the air nozzles in air grid 14, with each feed shroud 36 being aimed directly at its corresponding air nozzle to promote rapid and thorough mixing of the gases passing out of these two different nozzles. See, U.S. 4,801,731.

Although propylene/ammonia feed systems of this general type work well, they do suffer certain disadvantages. For example, because of constant exposure to ammonia at high temperature, the metal forming feed sparger 16 undergoes nitriding over time. As a result, individual sections of feed sparger 16, and sometimes the entire feed sparger, need to be replaced from time to time. This can be very expensive, especially since the reactor must be completely shut down while this is being done.

A second problem associated with this type of propylene/ammonia feed system is nonuniformity of operation. This not only adversely affects the productivity of the system but also contributes to non-uniform nitriding, which further exacerbates the nitriding problem.

### Summary

In accordance with this invention, a process according to claim 1 is provided which substantially reduces these problems, and in some instances eliminates them substantially completely.

In accordance with one feature of the sparger design, a gas-tight quick-disconnect fitting is used to attach the main header conduit of the sparger to the wall of the reactor through which the main header conduit passes, or to connect the various conduits forming the feed sparger to one another, or both. As a result of this feature, the time and labor needed to replace some or all of the feed sparger, when this becomes necessary due to excessive nitriding, is reduced substantially.

In accordance with another feature of the sparger design, the relative diameters of feed nozzles 34 are increased slightly as the travel path from the inlet of the feed sparger to each feed nozzle increases. As a result of this feature, the mass flow rate of the ammonia containing feed mixture passing through each feed nozzle is made more nearly uniform from feed nozzle to feed nozzle. This, in turn, leads to more nearly uniform operation from region to region inside the reactor, which enables productivity to be maximized. This feature also minimizes catalyst back up, *i.e*., contamination of the feed sparger with catalyst during start-up, shut-down and even normal operation, by ensuring a proper flow rate of gasses through the sparger feed nozzles at all times.

In accordance with a further feature of the sparger design, the diameters of laterals 32 are decreased from their proximal ends to their distal ends, *i.e.,* from their ends connected to the main header conduit to their ends remote from the main header conduit. As a result of this feature, the velocity of the ammonia containing feed mixture flowing through these laterals is kept high enough along their entire lengths, and especially at their distal ends, to sweep any ammoxidation catalyst that may be present to the next feed nozzle 34 for discharge from the lateral interior through this feed nozzle.

In accordance with the invention, the feed sparger 16 is subdivided into multiple feed sparger sections, each of which has its own inlet port for receiving an ammonia containing feed from outside the reactor. As a result of this feature, better control of the reactor from region to region can be achieved, because a separate control system can be used to monitor and control operations in each feed sparger section individually. Further, this disclosure describes a sparger for use in supplying an ammonia containing feed mixture from outside an ammoxidation reactor, through a reactor wall of the reactor and into a fluidized bed of ammoxidation catalyst inside the reactor, the sparger comprising a main header conduit, a sparger inlet in fluid communication with the main header conduit, the sparger inlet being rigidly attached to the reactor wall, and multiple lateral sparger conduits in fluid communication with the main header sparger conduit, the lateral sparger conduits defining feed nozzles for discharging propylene/ammonia feed mixture into the fluidized bed of ammoxidation catalyst, wherein the sparger inlet is rigidly attached to the reactor wall by means of a gas-tight quick-disconnect fitting.

In another embodiment, this disclosure describes a sparger for use in supplying an ammonia containing feed mixture from outside an ammoxidation reactor, through a reactor wall of the reactor and into a fluidized bed of ammoxidation catalyst inside the reactor, the sparger comprising a main header conduit, a sparger inlet in fluid communication with the main header conduit, and multiple lateral sparger conduits in fluid communication with the main header sparger conduit, the lateral sparger conduits defining feed nozzles for discharging the ammonia containing feed mixture into the fluidized bed of ammoxidation catalyst, wherein at least some of the lateral sparger conduits are attached to the main header conduit by respective gas-tight quick-disconnect fittings.

In still another embodiment, this disclosure describes a sparger for use in supplying an ammonia containing feed mixture from outside an ammoxidation reactor, through a reactor wall of the reactor and into a fluidized bed of ammoxidation catalyst inside the reactor, the sparger comprising a main header conduit, a sparger inlet in fluid communication with the main header conduit, and multiple lateral sparger conduits in fluid communication with the main header sparger conduit, each lateral sparger conduit defining feed nozzles for discharging the ammonia containing feed mixture into the fluidized bed of ammoxidation catalyst, wherein the feed nozzles have at least two different sizes, with the smaller feed nozzles being located closer to the sparger inlet and the larger nozzles being located farther away from the sparger inlet as determined by the distance the propylene/ammonia feed mixture travels through the sparger from the sparger inlet to each nozzle.

In a further embodiment, this disclosure describes a sparger for use in supplying an ammonia feed mixture from outside an ammoxidation reactor, through a reactor wall of the reactor and into a fluidized bed of ammoxidation catalyst inside the reactor, the sparger comprising a main header conduit, a sparger inlet in fluid communication with the main header conduit, and multiple lateral sparger conduits, each lateral sparger conduit having a proximal end in fluid communication with the main header conduit and a distal end remote from the main header conduit, each lateral sparger conduit further defining feed nozzles for discharging the ammonia containing feed mixture into the fluidized bed of ammoxidation catalyst, wherein the diameters of at least some of the lateral sparger conduits decrease from their proximal ends to their distal ends. This invention provides a process using a sparger in supplying an ammonia containing feed mixture from outside an ammoxidation reactor, through a reactor wall of the reactor and into a fluidized bed of ammoxidation catalyst inside the reactor, the improved sparger comprising a main header conduit, a sparger inlet in fluid communication with the main header conduit, and multiple lateral sparger conduits in fluid communication with the main header sparger conduit, the lateral sparger conduits defining feed nozzles for discharging the ammonia containing feed mixture into the fluidized bed of ammoxidation catalyst, wherein the improved sparger is composed of multiple feed sparger sections arranged inside the reactor, each feed sparger section having its own sparger inlet for receiving the ammonia containing feed from outside the reactor, its own main header conduit and its own system of lateral sparger conduits.

### Brief Description of the Drawings

This invention may be better understood by reference to the following drawings wherein:
Fig. 1 is a schematic view showing the reactor section of a conventional ammoxidation reactor used for making acrylonitrile;
Fig. 2 is a plan view showing the underside of the conventional sparger system of the ammoxidation reactor of Fig. 1;
Fig. 3 is a cross sectional view taken on line 3-3 of Fig 2, Fig. 3 showing the feed nozzles and associated feed shrouds of the conventional sparger system of Fig 2;
Fig. 4 is a sectional view illustrating the manner in which the main header conduit of the feed sparger of a commercial ammoxidation reactor penetrates and is connected to the side wall of the reactor;
Fig. 5 is a sectional view similar to Fig. 4, illustrating one feature of this invention in which the main header conduit of the feed sparger penetrates and is connected to the side wall of the reactor by means of a gas-tight quick-disconnect coupling;
Fig. 6 is a side view of the gas-tight, quick-disconnect coupling of Fig. 5;

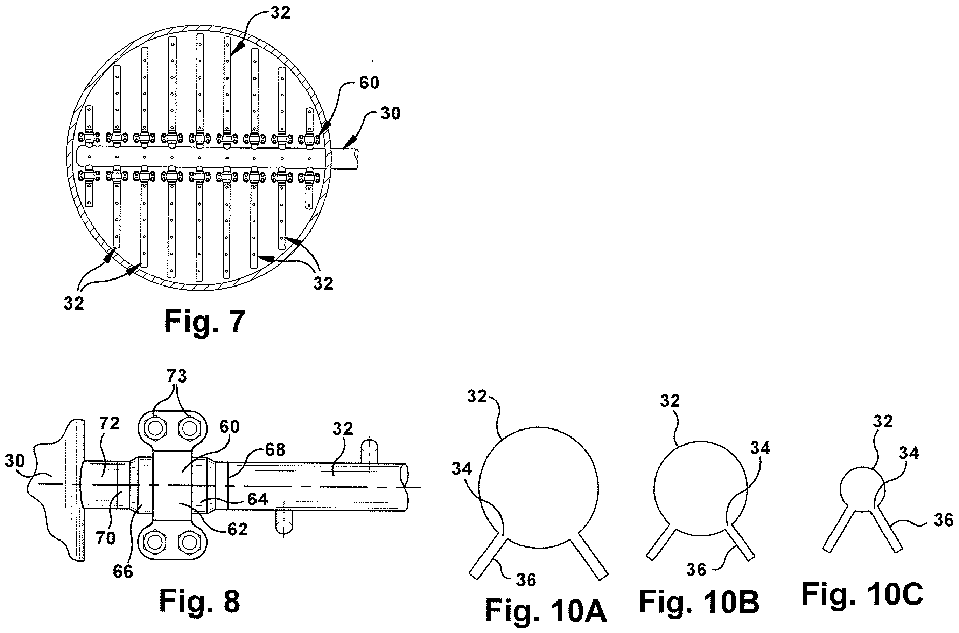

Fig. 7 is a cross-sectional view similar to Fig. 2, illustrating another feature of this invention in which the sparger lateral conduits are connected to the main header conduit of the sparger by means of gas-tight quick-disconnect couplings;
Fig. 8 is a plan view illustrating the gas-tight quick-disconnect couplings of Fig. 7 in more detail;

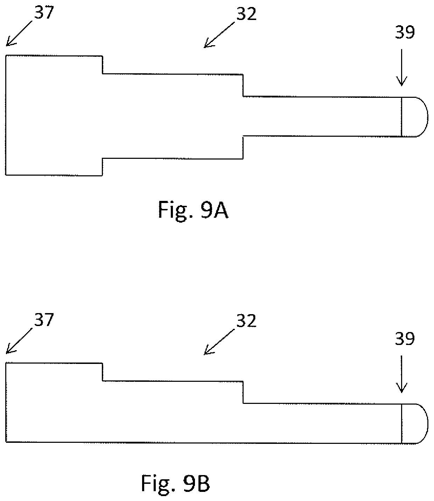

Figs. 9A and 9B are lateral cross sectional views of a sparger lateral conduit used in accordance with still another feature of this invention, illustrating how the diameter of this lateral decreases as the distance from the sparger header conduit increases;
Figs. 10A, 10B and 10C are transverse cross-sectional views of the sparger lateral conduit of Fig. 9 further illustrating how the diameter of this lateral decreases as the distance from the sparger header conduit increases;

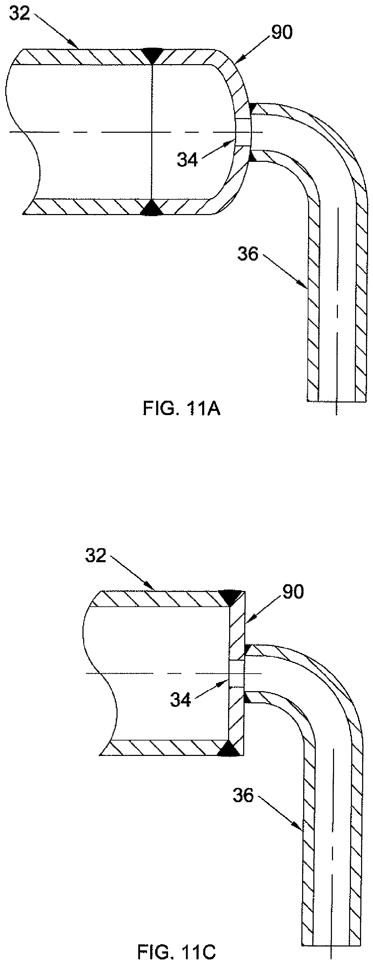

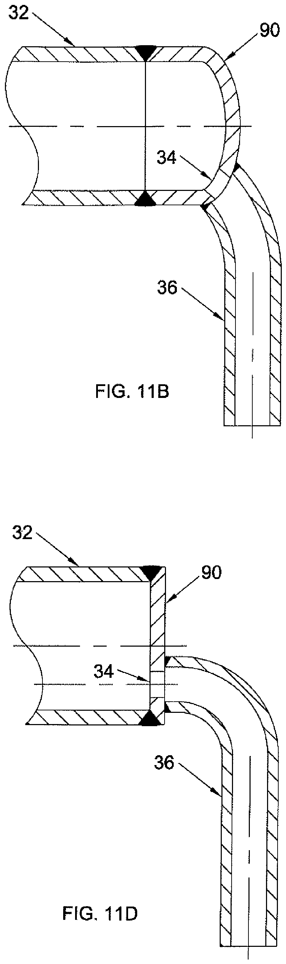

Figs. 11A, 11B, 11C and 11D are vertical cross-sectional of a sparger lateral end-cap used in accordance with still another feature of the inventive sparger system; and

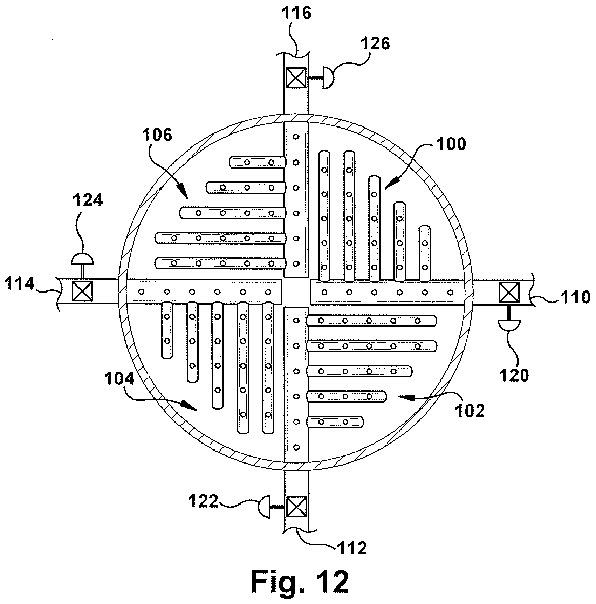

Fig. 12 is a plan view illustrating this invention in which the feed sparger of the acrylonitrile reactor is subdivided into multiple feed sparger sections.

### DETAILED DESCRIPTION

### Definitions

As used herein, "fluid communication" refers to a connection or conduit effective for allowing the same liquid or vapor to pass from one area to another.

As used herein, "releasably secured" refers to a non-welded connection that allows items to be disconnected by non-destructive means. For example, releasably secured may refer to bolts, fanged bolts, bolted flanges and combinations thereof.

As used herein, "ammonia containing feed mixture" refers to a blend of ammonia and saturated and/or unsaturated C3 to C4 hydrocarbons. Saturated and/or unsaturated C3 to C4 hydrocarbons may include propane, propylene, butane, butylene, and mixtures thereof.

### Quick Disconnect Couplings

As indicated above, a significant problem encountered in the operation of a commercial acrylonitrile reactor is the failure of the feed sparger over time due to nitriding of the metal from which it is formed. To solve this problem, it has already been proposed to make the sparger from nitride resistant alloys such as shown in U.S. 3,704,690, U.S. 4,401,153, U.S. 5,110,584 and EP 0 113 524. Unfortunately, this solution has proven to be unsuccessful for use in commercial acrylonitrile reactors due to certain problems which are unique to a fluidized bed catalytic ammoxidation reaction as well as reasons of cost.

Meanwhile, U.S. 5,256,810 describes a process for substantially eliminating nitriding of the sparger in a commercial acrylonitrile reactor by maintaining the temperature of the ammonia inside the sparger low enough to prevent nitriding from occurring through the use of a specially designed blanket of thermal insulation. However, this solution has also proven to be unsatisfactory because of cost and complicated design.

In accordance with this feature of the invention, this problem of sparger failure over time due to metal nitriding is dealt with by adopting a sparger design which enables individual sections of the sparger, as well as the entire sparger as a whole, to be replaced quickly and easily. Although it is still necessary to shut down the acrylonitrile reactor when this replacement is done, the time it takes to accomplish this replacement is shortened considerably with respect to conventional practice. As a result, the overall cost for dealing with this nitriding problem on a continuing basis, both in terms of lost production time as well as manpower costs, is reduced significantly.

Figs. 4, 5 and 6 illustrate one feature of this invention in which this sparger nitriding problem is dealt with by using a gas-tight, quick disconnect coupling to connect the inlet of the sparger system to the outer wall of the ammoxidation reactor. In the particular embodiment shown in these figures, an end of main header 30 is directly attached to wall 40 of reactor 10. In this design, therefore, this header end constitutes inlet 31 of sparger section 16. In other designs, intermediate piping can be used to connect sparger inlet 31 to header 30. For convenience, this feature of the invention will be described in connection the reactor design shown in Figs. 4, 5 and 6. However, it will be appreciated that this feature and its advantages are equally applicable to other reactor designs such as those, for example, in which sparger inlet 31 is separated from main header 30 by intermediate piping.

As shown in Fig. 4, the conventional way of attaching sparger inlet 31 of feed sparger 16 to the wall 40 of reactor 10 is by welding. Accordingly, when main header conduit 30 needs to be replaced, a welding repair approach must be adopted in which the portion of reactor wall 40 immediately surrounding main header conduit 30 is cut out by welding, the opening in reactor shell 12 formed thereby repaired by welding in a suitable patch, and a new main header conduit 30 installed in the repaired reactor wall 40 also by welding. This requires a significant amount of labor on-site, as well as additional materials, which can be expensive.

In accordance with this feature of the invention, this problem is avoided by adopting a gas-tight, quick disconnect coupling design for attaching main header conduit 30 to reactor wall 40. An example of such coupling is shown in Figs. 5 and 6, which shows "manhole" 42 in the form of a cylindrical sleeve 44, a first side of which is permanently welded in a gas-tight manner to periphery 46 of permanent opening 48 formed in reactor wall 40. The other or second side of cylindrical sleeve 44 carries flange 50, which defines a series of through holes for receiving bolts 52 therein. Meanwhile, collar 54 in the form of a flat circular plate is permanently welded in a gas-tight manner to the outside of main header conduit 30. In addition, collar 54 also defines a series of through holes 56 which correspond to the through holes in flange 50 of manhole 42.

With this structure, main header conduit 30 can be releasably secured to reactor wall 40 of reactor 10 in a gas-tight manner simply by bolting collar 54 of main header conduit 30 to flange 50 of manhole 42. In the same way, main header conduit 30 can be detached from reactor wall 40 simply by unbolting collar 54 from flange 50. Accordingly, replacement of an existing main header 30 which has become unusable due to excessive nitriding can be accomplished simply and easily through a simple unbolting and rebolting process. Because no on-site welding is required, this replacement procedure is much easier and less expensive to carry out than the welding repair approach which is conventionally done.

Figs. 2, 7 and 8 illustrate another feature of the invention in which gas-tight, quick-disconnect couplings are used to deal with the problem of sparger lateral nitriding. As shown in Fig. 2, the conventional way in which lateral conduits (or "laterals") 32 are attached to main header conduit (or "header") 30 is by welding. Accordingly, when individual laterals 32 need to be replaced due to excessive nitriding, a welding repair approach is adopted in which the old lateral are detached from main header conduit 30 by welding or other suitable cutting techniques and a new lateral attached to main header conduit 30 by welding. This also requires a significant amount of on-site labor, which is expensive.

In accordance with this feature of the invention, this problem is avoided by adopting a gas-tight, quick disconnect coupling design for attaching each lateral 32 to main header conduit 30. This is illustrated in Figs. 7 and 8, which show gas-tight, quick disconnect couplings 60 being used to connect each lateral 32 to main header conduit 30 of sparger system 16. Although these figures show each lateral being directly connected to main header conduit 30, it will be appreciated that one or more of these laterals can be indirectly connected to main header conduit 30 such as by means of intermediate piping (not shown).

Gas-tight, quick disconnect couplings 60 are couplings in which the mating parts, *i.e.,* the parts that come together when the connection is made and which move apart with the connection is broken, are specially designed to be jointed to one another by mechanical means only, *i.e.,* without welding or adhesive. Gas-tight, quick disconnect couplings are also designed to remain tightly sealed at high temperature conditions such as those encountered during normal operations of a typical commercial ammoxidation reactor, as well as during the temperature cycling that occurs when such a reactor is started up and shut down. An example of a commercially-available coupling which is suitable for this purpose is the Grayloc metal-to-metal bore seal clamp connectors available from Grayloc Products of Houston, Texas. Another example of a commercially-available coupling which is suitable for this purpose is the Techlok Clamp Connector available from the Vector group of Freudenberg Oil & Gas Technologies of Houston, Texas. Still another example of a commercially-available coupling which is suitable for this purpose is the G-Lok® Clamp Connector available from Australasian Fittings & Flanges of Osborne Park, WA, Australia. Conventional flanged connections are less desirable for this use, as they are prone to leakage due to temperature cycling during reactor operation.

Fig. 8 illustrates the structure of a typical gas-tight, quick disconnect coupling 60 including the manner in which it interconnects lateral 30 to main header conduit 30. As shown there, coupling 60 is formed from clamp assembly 62 which receives and holds together hubs 64 and 66, which are carried on the facing ends 68 and 70 of lateral 32 and header nipple 72. When secured in place by bolts 73, clamp assembly 62 causes a metal sealing ring (not shown) to be secured between, and to sealingly engage, hubs 64 and 66, thereby forming a gas-tight seal between lateral 32 and header 30.

By using gas-tight, quick disconnect couplings 60, each lateral 32 can be secured to and removed from main header conduit 30 simply by bolting or unbolting clamp assembly 62. Accordingly, replacement of an existing lateral 32 which has become unusable due to excessive nitriding can be accomplished simply and easily through a simple unbolting and rebolting process. Because no on-site welding is required, this replacement procedure is much easier and less expensive to carry out than the welding repair approach which is conventionally done.

The various aspects described herein may be utilized with reactors having various size diameters. In a preferred aspect, reactors may have external diameters from about 2 to about 12, in another aspect, about 5 to about 12 meters, in another aspect, about 8 to about 12 meters, and in another aspect, about 9 to about 11 meters.

### Variable Feed Nozzle Sizes

In accordance with another feature of this new sparger design, the diameters of feed nozzles 34 are increased slightly as the travel path from the inlet of the feed sparger to each feed nozzle increases.

When a ammonia containing feed mixture travels through sparger 16, heat transfer from the hot gases outside the sparger cause the temperature of the feed mixture inside the sparger to increase. As a result, the temperature of the feed mixture exiting each feed nozzle is different, depending on how long the feed mixture has been inside the sparger before exiting. Specifically, the temperature of the feed mixture exiting feed nozzles located farther away from the sparger inlet are hotter than the temperature of the feed mixture exiting feed nozzles located closer to the sparger inlet. In this context, "farther" and "closer" will be understood to mean farther and closer from the sparger inlet in terms of the length of the travel path beginning at the sparger inlet and ending at the particular feed nozzle through which the feed mixture exits the sparger.

In a conventional ammoxidation reactor, the diameters of feed nozzles 34 (Fig. 3) are all the same. As a result, the density of the feed mixture existing through feed nozzles 34 located farther away from the sparger inlet is less than the density of the feed mixture existing through feed nozzles 34 located closer to the sparger inlet, since density is inversely proportional to temperature. This, in turn, causes the mass flowrate of the ammonia containing feed mixture existing through feed nozzles 34 located farther away from the sparger inlet to be less than the mass flowrate of the feed mixture existing through feed nozzles 34 located closer to the sparger inlet, provided that the other conditions are the same, since mass flow rate is directly proportional to density. Unfortunately, this lack of uniformity in mass flow rate through each feed nozzle leads to less than optimal reactor performance as a whole, because the amount (*i.e.,* the total mass per unit time) of ammonia containing feed mixture entering bed 24 of ammoxidation catalyst in regions of the reactor where the feed nozzles are farther away from the sparger inlet is less than in regions where the feed nozzles are closer to the inlet.

In accordance with this feature of the invention, this problem is overcome by varying the size of sparger feed nozzles 34, with those feed nozzles located farther away from the sparger inlet being larger than those located closer to the sparger inlet. "Size," "larger" and "smaller" in this context refers to the cross-sectional area of the nozzle openings. In this aspect, a ratio of reactor external diameter to number of different size feed nozzles is about 0.5 to about 2.5, in another aspect, about 1 to about 2, and in another aspect, about 1.5 to about 2.

While nozzles of many different sizes can be used in a particular acrylonitrile reactor, it has been found that using nozzles of having from about 2 to about 10 different sizes, in another aspect, about 2 to about 8 different sizes, in another aspect, about 2 to about 6 different sizes, in another aspect, about 2 to about 4 different sizes, in another aspect, about 3 to about 6 different sizes, in another aspect, about 3 to about 4 different sizes, in another aspect, about 4 to about 8 different sizes, in another aspect, about 4 to about 6 different sizes, in another aspect, about 5 to about 6 different sizes , in another aspect, about 5 to about 7 different sizes, and in another aspect, about 5 to about 8 different sizes, depending on reactor diameter, is sufficient to overcome the above problem of non-uniform feeding in most acrylonitrile reactors. In another aspect, if the reactor has an external diameter of about 2 to about 5 meters then the feed nozzles have about 3 to about 4 different sizes. In another aspect, if the reactor has an external diameter of more than about 5 to about 12 meters then the feed nozzles have about 5 to about 8 different sizes. So, for example, using nozzles having three different sizes will normally be sufficient for "small" acrylonitrile reactors having diameters on the order of 8 to 12 feet (∼2.4 to ∼3.7 meters). On the other hand, using nozzles having five or six different sizes is more appropriate for "large" acrylonitrile reactors having diameters on the order of 26 to 32 feet (∼7.9 to ∼9.7 meters) or larger.

Generally speaking, the size (cross-sectional area) of feed nozzles 34 in a commercial acrylonitrile reactor range between 15 to 80 mm², more commonly 20 to 60 mm², depending on the size of the reactor and the density of the feed nozzles, *i.e.,* the number of feed nozzles 34 per square meter of reactor cross-section. This same nozzle sizing can also be used in connection with this feature of the invention. In other words, the average nozzle size of all feed nozzles in a given acrylonitrile reactor will correspond to these values.

In terms of the difference in nozzle size, the ratio of the biggest to the smallest nozzle in terms of the cross sectional area in a set of nozzles used for a particular ammoxidation reactor can be as small as 1.02 and as large as 1.35. The size of feed nozzles with intermediate sizes can easily be determined by calculation and/or routine experimentation.

In this regard, the purpose of using feed nozzles 34 of different sizes is to achieve a mass flow rate of feed mixture which is as nearly uniform as possible from feed nozzle to feed nozzle. Within a given sparger system, the mass flowrate of the feed mixture passing through any particular feed nozzle is based primarily on its density, which in turn is based primarily on its temperature. Accordingly, the particular sizes to use for particular nozzles of intermediate sizes can be easily determined by reference to the anticipated temperature of the feed mixture passing through that feed nozzle, which in turn can be easily determined either by actual measurement or by appropriate heat transfer calculations.

With this feature, the mass flow rate of the ammonia containing feed mixture passing through each feed nozzle is made more nearly uniform from feed nozzle to feed nozzle. This, in turn, leads to more nearly uniform operation from region to region inside the reactor, which enables productivity to be maximized. In this aspect, the mass flow rate through any one feed nozzles is within about 5% of a mass flow rate of any other nozzle, in another aspect, within about 4%, in another aspect, within about 3%, in another aspect, within about 2%, in another aspect, within about 1%, in another aspect, within about 0.5%, in another aspect, within about 0.25%, and within another aspect, within about 0.1%.

This feature also minimizes contamination of the feed sparger with catalyst during start-up, shut-down and even normal operation ("catalyst back up"), by ensuring a proper flow rate of gasses through the sparger feed nozzles at all times.

### Laterals with Decreasing Diameters

In accordance with still another feature of this new sparger design, the diameters of lateral sparger conduits or "laterals" 32 decrease from their proximal ends to their distal ends, *i.e.,* from their ends connected to header 30 to their opposite ends remote from header 30.

In a conventional acrylonitrile reactor, the diameters of lateral sparger conduits 32 are the same along the entire length of the conduit. With this design, the flow rate of the feed mixture through the conduit decreases substantially from its proximal end to its distal end, since much of the feed mixture entering the proximal end has exited the conduit through feed nozzles 34 located along its length. As a result, the velocity of the feed mixture inside these conduits at or near their distal ends is too slow to have a significant effect on any ammoxidation catalyst that may be present there.

In accordance with this feature of the invention, this problem is avoided by reducing the diameters of lateral sparger conduits or "laterals" 32 from their proximal ends to their distal ends. Figs. 9A, 9B, 10A, 10B and 10C illustrate this feature of the invention. As shown in these figures, the diameter of lateral 32 decreases in steps from its proximal end 37 to its distal end 39.

With this feature, the velocity of the ammonia containing feed mixture can be maintained high enough along its entire length to cause any ammoxidation catalyst that may have inadvertently contaminated the inside of sparger system 16 to be swept to the next feed nozzle 34, where it will be discharged along with the feed gas flowing through that feed nozzle. While this mechanism for catalyst removal is also used in earlier designs, the velocity of the feed gas at or near the distal ends of the laterals is too slow in these designs to sweep any catalyst present there to the next feed nozzle. In accordance with this feature of the invention, this problem is avoided by reducing the lateral's diameter from its proximal to its distal end. The result is that the velocity of the feed gas inside these lateral conduits remains high enough to sweep any catalyst that might be present there to the next available feed nozzle, even at the distal end of the conduit. Using a decreasing diameter enables a suitably high velocity even at the distal end of the conduit whilst also avoiding unacceptably high velocity and/or pressure drop at the proximal end of the conduits.

Although Figs. 9A, 9B, 10A, 10B and 10C show lateral 32 having three separate sections with different diameters, it should be appreciated than any convenient number of different diameters can be used in accordance with this invention. Generally speaking, the size and number of different diameters are selected to maintain a gas velocity of about 10 to 30, preferably 15 to 25, meters per second in all sparger conduits, *i.e.,* header 30 as well as all laterals 32.

The various aspects described herein may be utilized with reactors having various size diameters. In a preferred aspect, reactors may have external diameters from about 2 to about 12, in another aspect, about 5 to about 12 meters, in another aspect, about 8 to about 12 meters, and in another aspect, about 9 to about 11 meters.

### Lateral End-caps

In an optional yet preferred way of implementing the above feature of this invention, the distal ends 39 of lateral conduits 32 configured with decreasing diameters are terminated with end-caps penetrated by one or more feed nozzles 34 (*See,* Fig. 11). As mentioned above, this decreasing diameter feature ensures that velocity of feed gas flowing through laterals 32 at or near their distal ends remains relatively high. By terminating a lateral 32 having a smaller distal end 39 with an end-cap 90 including one or more feed nozzles, it is possible to ensure that this velocity remains high enough so that any ammoxidation catalyst that may be present at or near this distal end is kept moving so that it is eventually blown out of the lateral through a feed nozzles 34. Figs 11A and 11B show a rounded configuration, one with centrally located feed nozzle 34, and the other with a lowered feed nozzle 34. Figures 11C and 11D show a flat configuration, one with centrally located feed nozzle 34, and the other with a lowered feed nozzle 34. The lowered feed nozzle configurations minimize the dead space where catalyst could become trapped, but are potentially more expensive to fabricate.

### Multiple Feed Sparger Sections

In accordance with the invention, the feed sparger 16 is subdivided into multiple feed sparger sections, each of which has its own inlet port for receiving the ammonia containing feed from outside the reactor.

In a typical commercial ammoxidation reactor such as illustrated in Figs. 2, a single feed sparger system 16 is used in which a single horizontally oriented header 30 supplies all laterals 32 of the system. In most of these systems, as further illustrated in Figs. 2 and 4, the inlet 31 of sparger 16 is located in a side wall of reactor 10 in essentially the same horizontal plane as header 30.

When a sparger design of this type are used in larger acrylonitrile reactors, *i.e.,* reactors having diameters larger than about 6 meters (∼20 feet), the difference between the shortest and longest travel paths experienced by the ammonia containing feed gas in the sparger can get quite large since the feed gas enters only one end of the header 30 and hence must travel all the way to the other end for reaching the laterals attached there. As a result, the temperature, density and hence mass flowrate of the feed mixture exiting each feed nozzle 34 can vary considerably from feed nozzle to feed nozzle, depending on where it is located in the sparger system. As explained above, this variance in temperature, density and mass flowrate can cause considerable problems, both in terms of reactor performance as well as uniformity of nitriding.

To deal with this problem, it has already been proposed to move sparger inlet 31 to a location which is far above header 30 and to join sparger inlet 31 to the center of header 30 with suitable piping. The idea is that, because the feed gas is delivered to the center of header 30 rather than only one of its ends, the flow of this feed gas through header 30 to and through all laterals 32 will be more nearly uniform than would otherwise be the case. The problem with this approach, however, is that the additional piping that is necessary to connect sparger inlet 31 to the center of header 30 becomes nitrided over time, which is very disadvantageous for the reasons indicated above.

In accordance with the invention, feed sparger 16 is divided into multiple feed sparger sections in which each sparger subsection is provided with its own sparger inlet 31 for receiving ammonia containing feed from outside the reactor. Each sparger section is also provided with its own control system so that the flow of ammonia containing feed mixture in each sparger section can be separately controlled. In addition, sparger inlet 31 of each sparger section is located at or near the horizontal plane defined by header 30. Preferably, sparger inlet 31 of each sparger section is vertically spaced from this horizontal plane by no more than 3.048 m (10 feet), more preferably no more than 1.524 m (5 feet).

This feature of the inventive sparger design is illustrated in Fig. 12, which shows four separate and independent feed sparger sections 100, 102, 104 and 106, arranged inside the reactor in an essentially side by side relationship with respect to one another. In this context, "side by side" will be understood to mean that the individual sparger sections are arranged at essentially the same elevation inside the reactor as opposed to be arranged one atop the other. As further shown in Fig. 12, each of sparger sections 100, 102, 104 and 106 includes a sparger inlet 110, 112, 114 and 116, respectively, all of which are connected to a common feed header conduit (not shown) located outside reactor 10. In addition, separate control valves 120, 122, 124 and 126 connected to a control system (not shown) are provided.

With this feature, each separate sparger section can be separately controlled for adjusting the amount (mass flow rate) of the ammonia containing feed mixture being fed by that sparger section. This allows even better control of the reactor, as a whole, since each region of the reactor can be separately controlled. This, in turn, enables each region to be "tuned" to match the others, thereby achieving optimal performance across the reactor as a whole.

The various aspects described herein may be utilized with reactors having various size diameters. In a preferred aspect, reactors may have external diameters from about 2 to about 12, in another aspect, about 5 to about 12 meters, in another aspect, about 8 to about 12 meters, and in another aspect, about 9 to about 11 meters.

Although only a few specific examples of this invention have been described above, it should be apparent that many modifications can be made without departing from the scope of this invention. All such modifications are intended to be included within the scope of this invention, which is to be limited only by the following claims.

## Claims

1. A process for supplying an ammonia containing feed mixture to an ammoxidation reactor (10), the process comprising:
supplying an ammonia feed mixture from outside an ammoxidation reactor (10), through a reactor wall (40) of the reactor (10) and into a fluidized bed of ammoxidation catalyst (24) inside the reactor (10) through a sparger (16),
wherein the sparger (16) includes a main header conduit (30), a sparger inlet (31) in fluid communication with the main header conduit (30), and multiple lateral sparger conduits (32) in fluid communication with the main header sparger conduit (30), the lateral sparger conduits (32) defining feed nozzles (34) for discharging the ammonia containing feed mixture into the fluidized bed of ammoxidation catalyst (24), wherein the sparger is composed of multiple feed sparger sections (100, 102, 104, 106) arranged inside the reactor (10) in an essentially side by side relationship with respect to one another, each feed sparger section having its own sparger inlet (110, 112, 114, 116) for receiving ammonia containing feed from outside the reactor (40), its own main header conduit and its own system of lateral sparger conduits.

2. The process of claim 1, further comprising a common feed header conduit located outside the reactor, the inlet port of each feed sparger section being in fluid communication with the common feed header, each sparger section further including a control valve for controlling the flow of propylene/ammonia feed mixture into that feed sparger section.

3. The process of claim 1, wherein the main header conduit of each sparger section defines a horizontal plane, and further wherein the sparger inlet of each sparger section is vertically spaced form the vertical plane defined by its respective main header conduit by no more than 3.048 meters (10 feet).

4. The process of claim 1, wherein the reactor external diameter is 2 to 12 meters.

5. The process of claim 1, wherein the reactor external diameter is 8 to 12 meters.

6. The process of claim 1, wherein the reactor external diameter is 9 to 11 meters

## Patentansprüche

1. Verfahren zur Einspeisung einer ammoniakhaltigen Eintragsmischung in einen Ammoxidationsreaktor (10), wobei das Verfahren Folgendes umfasst:
die Einspeisung einer Ammoniak-Eintragsmischung von außerhalb eines Ammoxidationsreaktors (10) durch eine Reaktorwand (40) des Reaktors (10) und in eine Wirbelschicht von Ammoxidationskatalysator (24) innerhalb des Reaktors (10) über einen Verteiler (16),
wobei der Verteiler (16) eine Hauptkopfleitung (30), einen Verteilereinlass (31), der sich in Fluidverbindung mit der Hauptkopfleitung (30) befindet, und mehrere seitliche Verteilerleitungen (32), die sich in Fluidverbindung mit der Hauptkopfverteilerleitung (30) befinden, einschließt, wobei die seitlichen Verteilerleitungen (32) Eintragsdüsen (34) zum Ausbringen der ammoniakhaltigen Eintragsmischung in die Wirbelschicht von Ammoxidationskatalysator (24) definieren, wobei der Verteiler aus mehreren Eintragsverteilerabschnitten (100, 102, 104, 106) besteht, die im Reaktor (10) im Wesentlichen Seite an Seite zueinander angeordnet sind, wobei die Eintragsverteilerabschnitte jeweils über ihren eigenen Verteilereinlass (110, 112, 114, 116) zum Erhalt von ammoniakhaltigem Eintrag von der Außenseite des Reaktors (40), ihre eigene Hauptkopfleitung und ihr eigenes System seitlicher Verteilerleitungen verfügen.

2. Verfahren nach Anspruch 1, weiterhin umfassend eine außerhalb des Reaktors befindliche gemeinsame Eintragskopfleitung, wobei sich der Einlassanschluss jedes Eintragsverteilerabschnitts in Fluidverbindung mit dem gemeinsamen Eintragskopf befindet, wobei die Verteilerabschnitte jeweils weiterhin über ein Steuerungsventil zur Steuerung des Propylen/Ammoniak-Eintragsmischungsstroms in den betreffenden Eintragsverteilerabschnitt verfügen.

3. Verfahren nach Anspruch 1, wobei die Hauptkopfleitung jedes Verteilerabschnitts eine horizontale Ebene definiert und wobei weiterhin der Verteilereinlass jedes Verteilerabschnitts nicht mehr als 3,048 Meter (10 Fuß) von der durch seine jeweilige Hauptkopfleitung definierten vertikalen Ebene vertikal beabstandet ist.

4. Verfahren nach Anspruch 1, wobei der äußere Durchmesser des Reaktors 2 bis 12 Meter beträgt.

5. Verfahren nach Anspruch 1, wobei der äußere Durchmesser des Reaktors 8 bis 12 Meter beträgt.

6. Verfahren nach Anspruch 1, wobei der äußere Durchmesser des Reaktors 9 bis 11 Meter beträgt.

## Revendications

1. Procédé de distribution d'un mélange d'alimentation contenant de l'ammoniac dans un réacteur d'ammoxydation (10), le procédé comprenant :
la distribution d'un mélange d'alimentation d'ammoniac depuis l'extérieur d'un réacteur d'ammoxydation (10), à travers une paroi de réacteur (40) du réacteur (10) et dans un lit fluidisé de catalyseur d'ammoxydation (24) à l'intérieur du réacteur (10) par l'intermédiaire d'un pulvérisateur (16),
dans lequel le pulvérisateur (16) comprend un conduit de collecteur principal (30), une entrée de pulvérisateur (31) en communication fluidique avec le conduit de collecteur principal (30), et des conduits de pulvérisateur latéraux multiples (32) en communication fluidique avec le conduit de pulvérisateur de collecteur principal (30), les conduits de pulvérisateur latéraux (32) définissant des buses d'alimentation (34) pour décharger le mélange d'alimentation contenant de l'ammoniac dans le lit fluidisé de catalyseur d'ammoxydation (24), dans lequel le pulvérisateur est composé de sections de pulvérisateur d'alimentation multiples (100, 102, 104, 106) agencées à l'intérieur du réacteur (10) dans une relation essentiellement côte à côte les unes par rapport aux autres, chaque section de pulvérisateur d'alimentation ayant sa propre entrée de pulvérisateur (110, 112, 114, 116) pour recevoir une alimentation contenant de l'ammoniac depuis l'extérieur du réacteur (40), son propre conduit de collecteur principal et son propre système de conduits de pulvérisateur latéraux.

2. Procédé de la revendication 1, comprenant en outre un conduit de collecteur d'alimentation commun à l'extérieur du réacteur, un orifice d'entrée de chaque section de pulvérisateur d'alimentation est en communication fluidique avec le collecteur d'alimentation commun, chaque section de pulvérisateur comprenant une vanne de commande pour réguler le débit de mélange d'alimentation de propylène / ammoniac dans cette section de pulvérisateur d'alimentation.

3. Procédé de la revendication 1, dans lequel le conduit de collecteur principal de chaque section de pulvérisateur définit un plan horizontal, et en outre dans lequel l'entrée de pulvérisateur de chaque section de pulvérisateur est verticalement espacée par rapport au plan vertical défini par son conduit de collecteur principal respectif de pas plus de 3,048 mètres (10 pieds).

4. Procédé de la revendication 1, dans lequel le diamètre externe du réacteur est de 2 à 12 mètres.

5. Procédé de la revendication 1, dans lequel le diamètre externe du réacteur est de 8 à 12 mètres.

6. Procédé de la revendication 1, dans lequel le diamètre externe du réacteur est de 9 à 11 mètres.
